# EUROPÄISCHE PATENTSCHRIFT

(11) **EP 4 126 155 B1**
(45) Veröffentlichungstag und Bekanntmachung des Hinweises auf die Patenterteilung: **13.03.2024**
(21) Anmeldenummer: 20746162.5
(22) Anmeldetag: 23.07.2020
(51) Int. Cl.: A61M 16/14, A61M 16/16, A61M 16/10, A61M 16/00, G01N 27/22, A61M 16/04

(54) **ATEMGAS-ANFEUCHTUNGSSYSTEM**
RESPIRATORY GAS HUMIDIFICATION SYSTEM
SYSTÈME D'HUMIDIFICATION DE GZ RESPIRATOIRE

(43) Veröffentlichungstag der Anmeldung: 08.02.2023
(73) Patentinhaber: WILAmed GmbH, 91126 Kammerstein (DE)
(72) Erfinder: KLINGER, Miriam, 91126 Schwabach (DE)
(74) Vertreter: Stippl Patentanwälte
(86) Internationale Anmeldenummer: PCT/EP2020/070751
(87) Internationale Veröffentlichungsnummer: WO 2022/017601

(56) Entgegenhaltungen:
- WO-A1-2015/196379
- WO-A1-2018/172563
- US-A1- 2018 250 490
- US-A1- 2019 344 038

## Beschreibung

Die vorliegende Anmeldung betrifft ein Atemgas-Anfeuchtungssystem zur Konditionierung von Atemgas bei der Beatmung von Patienten gemäß Anspruch 1.

### Technologischer Hintergrund

Patienten mit besonders schweren oder lebensbedrohlichen Verletzungen oder Krankheiten müssen intensivmedizinisch betreut werden. Insbesondere zur Versorgung von Patienten mit unzureichender oder ausgesetzter Spontanatmung werden Beatmungsgeräte verwendet. Diese steuern den Atemgasfluss hin zu und weg von einem Patienten. Als Atemgas kann entweder normale Raumluft oder ein spezielles Sauerstoff-Luft-Gemisch verwendet werden.

Das Atemgas muss, bevor es in die Lunge des Patienten gelangt, konditioniert werden. Diese Atemgaskonditionierung kann entweder natürlich stattfinden oder künstlich durchgeführt werden, wobei bei beiden Atemgaskonditionierungen eine Temperatur von 37 °C und eine relative Feuchte von 100 % in der Lunge vorliegen müssen. Erreicht das Atemgas diese Werte nicht, kann eine Keimbesiedlung innerhalb der Atemorgane des Patienten deutlich erleichtert werden. Bei intubierten Patienten ist die natürliche Atemgaskonditionierung nicht möglich, sodass diese künstlich von Atemgasbefeuchtern durchgeführt werden muss, sodass eine Temperatur von ca. 37 °C und eine relative Feuchte von ca. 100 % vorliegt. Bei nicht intubierten Patienten können diese Werte auch niedriger sein, da eine natürliche Konditionierung des Atemgases stattfindet. Für die Konditionierung von Atemgas durch Atemgasbefeuchter wird in einer Befeuchtungskammer eine bestimmte Wassermenge von einer Heizplatte erhitzt und verdampft. Die Befeuchtungskammer ist an die Atemgaszufuhr des Patienten angeschlossen. Der Wasserdampf wird bei der Einatmung des Patienten mit dem Luftstrom in Richtung des Patienten transportiert und von diesem eingeatmet.

Die Funktionen des Atemgasbefeuchters können allerdings nicht durch das Beatmungsgerät gesteuert werden. Somit kann die Leistung des Atemgasbefeuchters nicht an die individuelle Atemdauer und -intensität des Patienten angepasst werden. Dadurch muss dieser selbstständig, beispielsweise durch eine Luftstrommessung in der Befeuchtungskammer, auf die Atmung des Patienten oder des Beatmungsgerätes reagieren. Die Steuerung des Atemgasbefeuchters benötigt allerdings Zeit, um die Verdampfungseinheit zu aktivieren. Folglich wird beim Beginn der Einatmung zu wenig Atemgas konditioniert und nicht-konditioniertes Atemgas vom Patienten eingeatmet. Nach der Beendigung der Einatmung kommt es zu einer verzögerten Abschaltung des Atemgasbefeuchters und es verbleibt zu viel Wasserdampf in der Befeuchtungskammer. Dies hat eine bedarfsunabhängige Konditionierung des Atemgases zur Folge und begünstigt eine Kondensation von Wasserdampf in der Atemgaszufuhr. Alternativ kann eine Zielmenge an Wasserdampf pro Zeiteinheit von medizinischem Personal eingestellt werden und der Wasserdampf wird kontinuierlich produziert. Dadurch findet mitunter eine Überproduktion an Wasserdampf während der Ausatmung des Patienten statt. Durch diese Überproduktion kann der Wasserdampf an den Wänden des Beatmungsschlauches kondensieren und eine Keimansiedlung innerhalb des Beatmungsschlauches begünstigt werden.

### Druckschriftlicher Stand der Technik

Eine Inline-Verdampfungseinrichtung ist aus der EP 2 329 977 B1 bekannt, welche direkt in einer Trägergasleitung positioniert ist und eine Flüssigkeit verdampft. Die Flüssigkeit wird aus einem Reservoir innerhalb oder außerhalb der Trägergasleitung mit einer Pumpe zu einer Verdampfungseinrichtung mit einem Heizelement gepumpt. Ein Sensor innerhalb der Trägergasleitung misst die Temperatur, den Durchfluss und/oder den Dampfgehalt der Trägergasmischung und gibt diese Daten an die Steuereinrichtung weiter. Eine Steuereinrichtung steuert mit diesen Daten die Verdampfungsrate der Inline-Verdampfungseinrichtung, indem sie die Leistung der Pumpe, die Temperatur des Heizelementes und den Durchfluss des Trägergases an von einem Bediener eingestellte Werte anpasst. Der Sensor kann Temperaturschwankungen innerhalb des Trägergassystems erfassen, welche bei der Atmung des Patienten entstehen. Bei beginnender Einatmung des Patienten erhöht sich der Luftstrom, wodurch die Temperatur absinkt. Bei der Ausatmung erhöht sich die Temperatur aufgrund des niedrigeren Luftstroms. Mit diesen Temperaturschwankungen bestimmt die Steuereinrichtung die Atemtätigkeit des Patienten. Die Steuerung berechnet aus den historischen Respirationsdaten statistisch, wann die nächste Inspiration des Patienten wahrscheinlich erfolgt. Durch diese Antizipation kann die Inline-Verdampfungseinrichtung ihre Verdampfungsleistung anpassen, wenn eine neue Inspiration des Patienten am wahrscheinlichsten ist. Die Atemtätigkeit des Patienten wird somit nur indirekt über Temperaturschwankungen innerhalb der Trägergasleitung gemessen. Die Inline-Verdampfungseinrichtung ist zwischen 15 - 45 cm entfernt vom Patienten positioniert.

Aus der EP 2 269 680 B1 ist ein System zum Anfeuchten von Atemgasen bekannt. Hierbei wird von einem Arzt eine definierte Menge an Wasser eingestellt, welche einer Verdampfungskammer über ein präzise gesteuertes Dosiersystem zugeleitet wird. Dort wird das Wasser kontinuierlich verdampft. Der Wasserdampf wird im Anschluss zu einem Feuchtigkeitstauscher geleitet, wo der Wasserdampf gesammelt wird. Bei der Inspiration eines Patienten strömt das Atemgas durch den Feuchtigkeitstauscher und nimmt die dort akkumulierte Feuchtigkeit auf.

DE 697 28 819 T2 beschreibt eine Einrichtung zur Behandlung von Atemgas eines intubierten Patienten. Hierbei wird eine bestimmte Menge an Wasser dem Atemgas für die Inhalation des Patienten zugeführt. Die Wassermenge wird anhand der davorliegenden, einzelnen Exhalation bestimmt. Die bestimmte Wassermenge wird von einem Kolben in einen Zylinder gesaugt und bei der Inhalation des Patienten direkt in den Intubationsschlauch gesprüht. Die Befeuchtung des Atemgases findet möglichst patientennah an der patientenseitigen Öffnung des Intubationsschlauches statt.

Die DE 10 2006 045 739 B3 offenbart eine Vorrichtung mit einem Beatmungsgerät und einem Anfeuchter. Hierbei ist eine bidirektionale Datenübertragung zwischen dem Beatmungsgerät und dem Anfeuchter vorgesehen, sodass beide Geräte Daten untereinander austauschen können. Dies können unter anderem Daten zur Sauerstoffkonzentration und zu den Gebläseeinstellungen des Beatmungsgerätes sein. Der Datenaustausch ermöglicht, dass der Anfeuchter bei deaktiviertem Gasfluss vom Beatmungsgerät zum Patienten deaktiviert wird. Dadurch kann eine Auskondensation von Wasser im Einatemschlauch verhindert werden. Zusätzlich kann die Befeuchtungsleistung des Anfeuchters während der Inspiration des Patienten an den Gasfluss zum Patienten angepasst werden.

Die WO 2018/172563 A1 offenbart ein Aerosol-Verabreichungssystem zur Verabreichung eines einatembaren aerosolförmigen Medikamentes. Hierbei wird das flüssige Medikament mittels eines Vibrationselementes aerolisiert. Des Weiteren sind Sensoren vorgesehen, welche die Atemtätigkeit eines Patienten überwachen und den Durchfluss von Atemgas messen.

Die US 2018/250490 A1 beschreibt ein Atemgas-Anfeuchtungssystem, welches Flüssigkeit mit Hilfe eines Heizelementes verdampft. Das Atemgas-Anfeuchtungssystem wird von einem Steuerungsgerät gesteuert, welches hierfür auf verschiedene Sensoren und Messdaten, z. B. Messdaten des Beatmungsgerätes, zugreift.

Die WO 2015/196379 A1 beschreibt einen Mikro-Befeuchter mit einer Zerstäuberplatte. Diese zerstäubt eine in einer Flüssigkeitskammer befindliche Flüssigkeit mittels Vibrationen. Zudem ist ein Sensor vorgesehen, welcher am Y-Stück des Atemschlauches angeordnet ist.

Die US 2019/344038 A1 offenbart einen Befeuchter, welcher Flüssigkeit mittels eines Zerstäubers in einen Strömungsweg sprüht. Der Befeuchter beinhaltet ein Steuerungsgerät, welches einen Einatemflussbefehl von einem Beatmungsgerät erhalten kann. Zudem ist ein Temperatur- und/oder Feuchtigkeitssensor am Y-Stück angeordnet.

### Aufgabe der vorliegenden Erfindung

Die Aufgabe der vorliegenden Erfindung besteht darin, ein Atemgas-Anfeuchtungssystem zur Verfügung zu stellen, welches eine an die aktuelle und individuelle Atemtätigkeit eines Patienten angepasste Konditionierung von Atemgas ermöglicht.

### Lösung der Aufgabe

Die vorstehende Aufgabe wird bei dem Atemgas-Anfeuchtungssystem durch die Merkmale des Anspruchs 1 gelöst. Zweckmäßige Ausgestaltungen der Erfindung werden in den abhängigen Ansprüchen beansprucht.

Erfindungsgemäß umfasst das Atemgas-Anfeuchtungssystem ein erstes Sensorsystem, welches einen Sensor für eine Information zur Spontanatmung eines Patienten umfasst oder ein Beatmungsgerät eine Information zur Atemtätigkeit des Patienten erzeugt und eine Pumpe in Abhängigkeit der Information eine Fluidmenge dosiert, welche von einem Verdampfer verdampft wird. Die Fluidmenge korreliert somit mit der Atemtätigkeit des Patienten. Die Information kann beispielsweise ein digitales Signal oder ein analoges Signal oder Daten oder eine Messgröße sein, welche die Atemtätigkeit des Patienten oder die Beatmungstätigkeit des Beatmungsgerätes, z. B. die Atmungstiefe und/oder -dauer, wiedergibt. Dadurch kann die Leistung des Atemgas-Anfeuchtungssystems durch ein Steuergerät an die aktuelle und individuelle Atemtätigkeit des Patienten angepasst werden. Mithin findet eine bedarfsgerechte Konditionierung des Atemgases statt. Durch die bedarfsgerechte Konditionierung des Atemgases wird eine Übersättigung des Atemgases vermieden und die Gefahr einer Kondensation des Fluiddampfes innerhalb der Atemgas-Zufuhr zum Patienten kann reduziert werden. Somit atmet der Patient optimal konditioniertes Atemgas ein und es kann eine optimale Versorgung des Patienten während der Beatmung sichergestellt werden. Durch das bedarfsgerecht konditionierte Atemgas werden die Reinigungsmechanismen des Atmungstraktes, z. B. die mukoziliäre Clearance, gefördert und die Gefahr eine Keimbesiedlung innerhalb des Atmungstraktes wird reduziert. Vorzugsweise ist das Atemgas-Anfeuchtungssystem patientennah positioniert. Dadurch wird die Strecke, welche das konditionierte Atemgas zurücklegen muss, erheblich reduziert und eine Kondensation des Fluiddampfes kann effektiv vermieden werden.

Vorteilhafterweise ist der Sensor direkt am Patienten angebracht. Dadurch wird die Information direkt am Patienten aufgenommen, wodurch sie die Atemtätigkeit des Patienten besonders präzise wiedergibt. Dies hat den Vorteil, dass das Atemgas-Anfeuchtungssystem direkt auf die aktuelle und individuelle Atemtätigkeit des Patienten reagieren und das Atemgas bedarfsgerecht konditionieren kann.

Dadurch, dass der Sensor ein Abdominalsensor und/oder ein Zwerchfellsensor sein kann, kann über eine Bewegung des Abdomens und/oder des Zwerchfells die aktuelle und individuelle Atemtätigkeit des Patienten bestimmt werden. Daraus resultiert der Vorteil, dass die Atemtätigkeit des Patienten besonders einfach und direkt gemessen werden kann.

Vorteilhafterweise erkennt die Steuerungseinheit, ob sie die Information von dem Beatmungsgerät oder von dem Sensor erhält. Demnach kann das Atemgas-Anfeuchtungssystem auch für Patienten verwendet werden, welche keine Spontanatmung mehr aufweisen und künstlich beatmet werden müssen. Bei Patienten mit unruhiger Spontanatmung und mit teilweise unterstützender künstlicher Beatmung kann das Atemgas-Anfeuchtungssystem verwendet werden, da es automatisch auf die Information zurückgreift, welche die aktuellen Informationen über die Atem- oder Beatmungstätigkeit enthält.

Indem die Steuerungseinheit aus der Information ein Dampfvolumen bestimmen kann, welches zur bedarfsgerechten Konditionierung des Atemgases benötigt wird, kann sichergestellt werden, dass das erzeugte Dampfvolumen zur Konditionierung des Atemgases mit der aktuellen und individuellen Atemtätigkeit des Patienten korreliert. Dadurch findet eine optimale Konditionierung des Atemgases statt und eine Übersättigung des Atemgases mit Fluiddampf sowie eine Kondensation des Fluids werden effektiv vermieden.

Vorteilhafterweise wird die Pumpe von der Steuerungseinheit in der Art angesteuert, dass die Pumpe dem Verdampfer eine bestimmte Menge an Fluid zuleitet, welches erforderlich ist, um das benötigte Dampfvolumen durch den Verdampfer zu erzeugen. Hierdurch wird sichergestellt, dass der Verdampfer die richtige Menge an Fluid erhält.

Zweckmäßigerweise verarbeitet die Steuerungseinheit die Information des Beatmungsgerätes oder des Sensors in Echtzeit und steuert das Atemgas-Anfeuchtungssystem in Echtzeit. Dadurch wird ein Echtzeitbetrieb des Atemgas-Anfeuchtungssystems sichergestellt und eine sofortige Reaktion des Atemgas-Anfeuchtungssystems auf die Atemtätigkeit des Patienten kann gewährleistet werden. Das Atemgas wird dementsprechend in Echtzeit bedarfsgerecht konditioniert. Insbesondere ist dadurch auch eine schnelle Reaktion auf eine unruhige Atemtätigkeit des Patienten möglich. Durch die Echtzeitsteuerung wird eine verzögerte Konditionierung des Atemgases vermieden. Demzufolge wird die Versorgung des Patienten deutlich verbessert.

Vorteilhafterweise sind die Pumpe und der Verdampfer nur während der Einatmung des Patienten aktiv. Dadurch wird während der Ausatmung des Patienten kein Fluid verdampft und eine Übersättigung des Atemgases innerhalb der Atemgaszufuhr wird effektiv vermieden.

Indem der Verdampfer das Fluid, welches ihm durch die Pumpe zugeleitet wird, erfindungsgemäß vollständig verdampft, verbleibt erfindungsgemäß kein Fluid im Verdampfer. Dies verhindert, dass sich nicht verdampftes Fluid innerhalb des Verdampfers ansammelt, wodurch im nächsten Einatmungszug zu viel Fluid verdampft werden könnte. In diesem Fall würde eine zu große Menge an Fluiddampf entstehen und das Atemgas würde unzureichend konditioniert bzw. übersättigt werden. Folglich könnte der Fluiddampf innerhalb der Atemgas-Zufuhr kondensieren.

Erfindungsgemäß umfasst der Verdampfer ein Heizelement, welches das zugeleitete Fluid verdampft.

Vorteilhafterweise kann das Heizelement als Peltier-Element ausgebildet sein. Peltier-Elemente zeichnen sich durch geringe Einbaumaße und eine exakte Ansteuerbarkeit aus.

Vorteilhaferweise kann das Atemgas-Anfeuchtungssystem ein zweites Sensorsystem mit mindestens einem Sensor, vorzugsweise mehreren Sensoren, umfassen, wobei der Sensor bzw. die Sensoren innerhalb der zum Patienten führenden Atemgaszufuhr vor und/oder nach dem Verdampfer positioniert ist bzw. sind.

Dadurch dass das zweite Sensorsystem einen Feuchtigkeitssensor umfasst, kann die Feuchte, vorzugsweise die relative Feuchte des Atemgases bestimmt werden. Dies ist vorteilhaft, um die Menge des verdampften Fluids an die Feuchte des eintreffenden Atemgases anzupassen, sodass dieses beim Verlassen des Verdampfers optimal konditioniert ist.

Indem das zweite Sensorsystem einen Temperatursensor umfasst, kann die Temperatur des zugeführten Atemgases besonders einfach bestimmt werden. Da die relative Feuchte von der Temperatur des Atemgases abhängt, kann über eine Temperaturmessung des Atemgases derjenige Fluidbedarf ermittelt werden, welcher benötigt wird, um die gewünschte Feuchte des Atemgases zu erreichen.

Dadurch, dass das zweite Sensorsystem einen Strömungssensor zur Bestimmung der Strömungsgeschwindigkeit des zugeführten Atemgases umfasst, kann die Leistung des Verdampfers besonders einfach an wechselnde Strömungsgeschwindigkeiten innerhalb der Atemgaszufuhr angepasst werden.

Vorteilhafterweise verwendet die Steuerungseinheit die ermittelten Sensordaten des zweiten Sensorsystems, um die Leistung des Atemgas-Anfeuchtungssystems zu steuern, sodass eingestellte Zielgrößen erreicht werden. Als Zielgrößen können beispielsweise die relative Feuchte und die Temperatur des Atemgases nach dem Verdampfer eingestellt werden. Indem die Sensordaten vor und/oder nach dem Verdampfer mit Zielgrößen verglichen werden, kann die Steuerungseinheit die Leistung des Atemgas-Anfeuchtungssystems kontinuierlich justieren, sodass die eingestellten Zielgrößen erreicht werden.

Bevorzugt verwendet die Steuerungseinheit zur Bestimmung des benötigten Dampfvolumens zusätzlich die Daten des zweiten Sensors. Dadurch kann das benötige Dampfvolumen besonders effektiv und genau bestimmt werden und an die Eigenschaften des Atemgases vor dem Verdampfer angepasst werden.

Vorteilhafterweise konditioniert das Atemgas-Anfeuchtungssystem das Atemgas auf eine relative Feuchte von 80 % bis 100 %, vorzugsweise von 90 % bis 100 %, besonders bevorzugt von 95 % bis 100 %.

Idealerweise konditioniert das Atemgas-Anfeuchtungssystem das Atemgas auf eine auf eine Temperatur von 33 °C bis 38 °C, vorzugsweise von 35 °C bis 37 °C, besonders bevorzugt von 36 °C bis 37 °C.

### Beschreibung der Erfindung anhand von Ausführungsbeispielen

Nachstehend werden zweckmäßige Ausgestaltungen der vorliegenden Erfindung näher beschrieben. Wiederkehrende Merkmale sind der Übersichtlichkeit halber lediglich mit einem Bezugszeichen versehen. Es zeigen:
- Fig. 1:: eine schematische Darstellung einer ersten Ausgestaltung des Atemgas-Anfeuchtungssystems gemäß der vorliegenden Erfindung;
- Fig. 2:: eine beispielhafte Darstellung der Funktionselemente des Computers des Atemgas-Anfeuchtungssystems gemäß Fig. 1.
- Fig. 3:: eine schematische Darstellung der Funktionselemente des Verdampfers des Atemgas-Anfeuchtungssystems gemäß Fig. 1;
- Fig. 4:: eine schematische Darstellung einer zweiten Ausgestaltung des Atemgas-Anfeuchtungssystems gemäß der vorliegenden Erfindung sowie
- Fig. 5:: eine beispielhafte Darstellung der Funktionselemente des Computers des Atemgas-Anfeuchtungssystems gemäß Fig. 4.

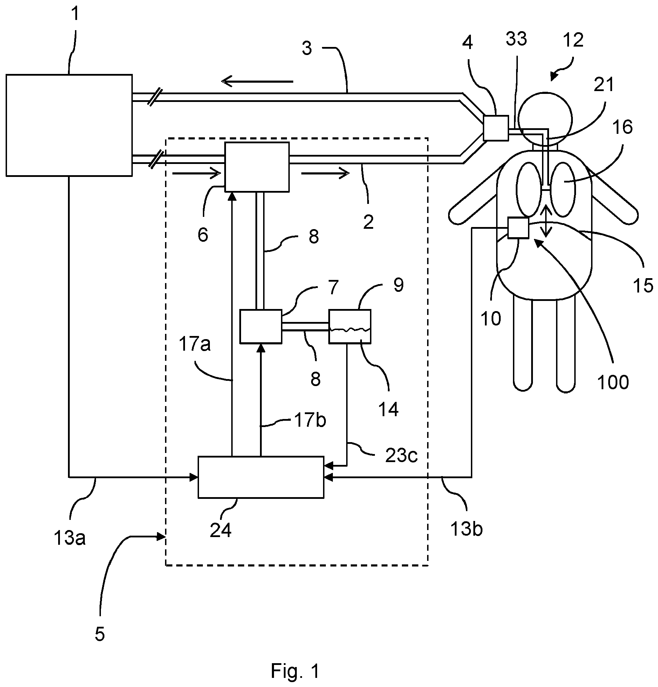

Bezugszeichen 5 in Fig. 1 bezeichnet das Atemgas-Anfeuchtungssystem gemäß der vorliegenden Erfindung in seiner Gesamtheit. Das Atemgas-Anfeuchtungssystem 5 dient der Konditionierung von Atemgas bei der Beatmung von Patienten 12. Hierfür wird neben dem Atemgas-Anfeuchtungssystem ein Beatmungsgerät 1, eine Atemgaszufuhr 2 und eine Atemgasrückfuhr 3 eingesetzt. Die Atemgaszufuhr 2 leitet das Atemgas vom Beatmungsgerät 1 hin zum Patienten 12. Die Atemgasrückfuhr 3 führt das ausgeatmete Atemgas des Patienten 12 zurück zu dem Beatmungsgerät 1. Die Atemgaszufuhr 2 und die Atemgasrückfuhr 3 werden über ein Y-Stück 4 zusammengeführt, an welchem ein Anschlussstück 33 befestigt ist. Das Anschlussstück 33 ist mit dem Patienten 12 verbunden, sodass der Patient 12 über dieses mit Atemgas versorgt wird. Das Y-Stück 4 sorgt dafür, dass ausgeatmetes Atemgas ausschließlich in die Atemgasrückfuhr 3 geleitet wird und das Atemgas während der Einatmung des Patienten 12 nur aus der Atemgaszufuhr 2 strömen kann. Das erfindungsgemäße Atemgas-Anfeuchtungssystem 5 wird in die Atemgaszufuhr 2 integriert und ist hierbei möglichst patientennah positioniert. Vorzugsweise ist das Atemgas-Anfeuchtungssystem 5 zwischen 10 cm und 50 cm vom Patienten 12 entfernt.

Falls der Patient 12 über Spontanatmung verfügt, kontrahiert das Zwerchfell 15 während der Einatmung und senkt sich ab. Dadurch entsteht ein Unterdruck im Brustraum, wodurch sich die Lunge 16 mit Atemgas füllt. Das Atemgas strömt hierbei vom Beatmungsgerät 1, über die Atemgaszufuhr 2, das Atemgas-Anfeuchtungssystem 5, das Y-Stück 4 und über das Anschlussstück 33 durch den Atemtrakt 21 des Patienten 12 in seine Lunge 16. Bei der Ausatmung entspannt sich das Zwerchfell 15, wobei es sich anhebt, die Lunge 16 zieht sich zusammen und das Atemgas strömt über das Mundstück 33, das Y-Stück 4 und die Atemgasrückfuhr 3 zurück zu dem Beatmungsgerät 1. Das Beatmungsgerät 1 kann bei der Spontanatmung des Patienten 12 die Atmung unterstützen.

Alternativ kann die Beatmung des Patienten 12 auch ausschließlich über das Beatmungsgerät 1 stattfinden, wenn der Patient 12 nicht mehr selbstständig atmet. Hierbei wird das Atemgas bei der Einatmung durch das Beatmungsgerät 1 in die Lunge 16 des Patienten 12 gedrückt und bei der Ausatmung aus der Lunge 16 des Patienten 12 gesaugt.

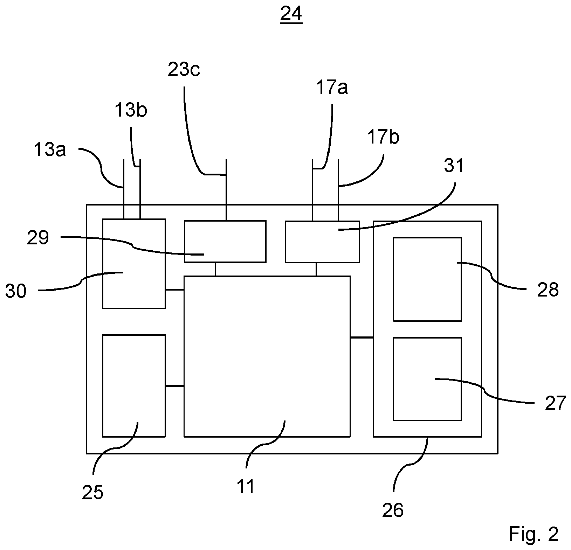

Das Atemgas-Anfeuchtungssystem 5 umfasst ein Fluid 14, vorzugsweise Wasser, welches in einem Fluidreservoir 9 bevorratet wird, einen Verdampfer 6, eine Pumpe 7 und einen Computer 24, welcher eine Steuerungseinheit 11 umfasst, siehe auch Fig. 2. Die Steuerungseinheit 11 steuert das Atemgas-Anfeuchtungssystem 5. Die Pumpe 7 pumpt eine definierte, an die Atemtätigkeit des Patienten 12 angepasste Menge an Fluid 14 vom Fluidreservoir 9 über Fluidleitungen 8 zu dem Verdampfer 6. Die Atemgaszufuhr 2 verläuft über den Verdampfer 6 oder durch den Verdampfer 6 hindurch. Die definierte Menge an Fluid 14 wird von dem Verdampfer 6 verdampft, sodass sich das Atemgas innerhalb der Atemgaszufuhr 2, welche durch den Verdampfer 6 verläuft, mit Feuchtigkeit anreichert und sich innerhalb der Atemgaszufuhr 2 ein Aerosol bildet. Ebenfalls kann das Atemgas durch den Verdampfer 6 erhitzt werden. Dadurch wird das Atemgas konditioniert. Wenn der Patient 12 einatmet bzw. beatmet wird, strömt das konditionierte Atemgas in die Lunge 16 des Patienten 12.

Erfindungsgemäß umfasst das Atemgas-Anfeuchtungssystem 5 ein erstes Sensorsystem 100 mit mindestens einem Sensor zur Ermittlung der Atemtätigkeit des Patienten 12. Das erste Sensorsystem 100 umfasst einen Sensor 10 für eine Information, welche beispielsweise die Spontanatmung des Patienten 12 hinsichtlich Respirationsdauer und/oder Respirationstiefe wiedergibt. Alternativ kann gemäß der Erfindung die Information auch von dem Beatmungsgerät 1 erzeugt werden. Die Information gibt hierbei z. B. die Beatmungsdauer und/oder Beatmungstiefe des Patienten 12 wieder. Erfindungsgemäß wird in Abhängigkeit der Information von der Pumpe 7 eine Fluidmenge dosiert, welche dem Verdampfer 6 zugeleitet wird und von diesem verdampft wird. Dadurch kann die Konditionierung des Atemgases an die aktuelle und individuelle Atemtätigkeit des Patienten 12 angepasst werden.

An dem Computer 24 werden eine Datenleitung 13a des Beatmungsgerätes 1 und eine Datenleitung 13b des Sensors 10 angeschlossen, wie Fig. 1 und 2 zeigen. Die Datenleitungen 13a, 13b dienen dazu, die Information an die Steuerungseinheit 11 zu übertragen. Zusätzlich werden eine Steuerungssignalleitung 17a zur Steuerung des Verdampfers 6 und eine Steuerungssignalleitung 17b zur Steuerung der Pumpe 7 sowie eine Messsignalleitung 23c angeschlossen. Die Messsignalleitung 23c dient beispielsweise der Übertragung der aktuellen Fluidmenge innerhalb des Fluidreservoirs 9. Die Informationsübertragung zwischen dem Computer 24 und dem Beatmungsgerät 1, dem Sensor 10 sowie dem Fluidreservoir 9 und weiteren Messsignalgebern kann sowohl kabelgebunden als auch drahtlos erfolgen.

Der Sensor 10 für die Information ist vorzugsweise direkt am Patienten 12 angebracht. Bei dem Sensor 10 kann es sich um einen Abdominalsensor und/oder einen Zwerchfellsensor handeln. Mit diesen Sensoren kann z. B. der Beginn, das Ende und/oder die Tiefe eines Atemzuges bestimmt werden und/oder erfasst werden, ob der Atemzug gleichmäßig oder ungleichmäßig ist. Mit diesen Sensoren kann insbesondere der Beginn der Einatmung festgestellt werden, bevor sich die Lunge 16 mit Atemgas füllt. Dadurch kann die Steuerung 11 des Atemgas-Anfeuchtungssystems 5 auf eine bevorstehende Einatmung reagieren und die Leistung des Atemgas-Anfeuchtungssystems 5 erhöhen, bevor Atemgas in die Lunge 16 des Patienten 12 strömt.

Die Funktionselemente des Computers 24 werden in Fig. 2 dargestellt. Die Datenleitungen 13a, 13b werden über eine Signalauswertung 30 mit der Steuerungseinheit 11 verbunden. Die Steuerungssignalleitungen 17a, 17b sind über einen Steuerungssignalausgang 31 mit der Steuerungseinheit 11 verbunden. Über eine Sensorschnittstelle 29 wird die Messsignalleitung 23c mit der Steuerungseinheit 11 verbunden. Alternativ oder zusätzlich können auch weitere Messsignalleitungen an der Sensorschnittstelle 29 angeschlossen werden, um weitere Statusinformationen des Atemgas-Anfeuchtungssystems 5 zu erhalten. Der Computer 24 wird mit einer Energieversorgung 25 mit Strom versorgt. Hierbei kann die Stromversorgung durch einen Netzanschluss, einen Akkumulator oder eine Batterie erfolgen. Ferner umfasst der Computer 24 eine Benutzerschnittstelle 26 mit einem Bedienelement 28 und einem Display 27.

Mit Hilfe des Bedienelementes 28 können Einstellungen am Atemgas-Anfeuchtungssystem 5 durchgeführt werden. Es können beispielsweise Zielgrößen eingestellt werden, welche das Atemgas durch das Atemgas-Anfeuchtungssystem 5 erreichen sollen, um bedarfsgerecht konditioniert zu werden. Entsprechende Zielgrößen des Atemgases können beispielsweise eine bestimmte Temperatur und/oder eine bestimmte Feuchte des Atemgases sein. Dadurch können beispielsweise die Temperatur und die Feuchte an den individuellen Bedarf des Patienten 12 angepasst werden können. So kann der Gehalt der Feuchte und Temperatur zwischen intubierten und nicht-intubierten Patienten unterschiedlich eingestellt werden. Dies verbessert die Versorgung des Patienten erheblich.

Auf dem Display 27 können z. B. Messwerte von unterschiedlichen Sensoren und Statusmeldungen des Atemgas-Anfeuchtungssystems 5 angezeigt werden. So kann beispielsweise der aktuelle Füllstand des Fluidreservoirs 9 angezeigt werden. Sollten Messwerte außerhalb eines festgelegten Bereichs liegen, so kann eine visuelle Warnmeldung erfolgen, beispielsweise wenn der Füllstand des Fluidreservoirs 9 niedrig ist. Diese Warnmeldung kann mit Hilfe eines (nicht in der Fig. dargestellten) Lautsprechers durch eine akustische Warnmeldung ergänzt oder ersetzt werden.

Durch die Signalauswertung 30 erhält die Steuerungseinheit 11 alle relevanten Daten der eintreffenden Information des Sensors 10 oder des Beatmungsgerätes 1. Dadurch kann die Steuerungseinheit 11 erkennen, ob sie die Information von dem Beatmungsgerät 1 oder dem Patienten 12 erhält.

Die Steuerungseinheit 11 kann aus der Information des Sensors 10 oder des Beatmungsgerätes 1 ein benötigtes Dampfvolumen bestimmen, welches benötigt wird, um das Atemgas zu konditionieren. Die Konditionierung des Atemgases wird hierbei an die aktuelle und individuelle Atemtätigkeit des Patienten 12 angepasst.

Basierend auf dem benötigten Dampfvolumen steuert die Steuerungseinheit 11 die Pumpe 7 in der Art, dass diese dem Verdampfer 7 eine Menge an Fluid 14 zuleitet, welche benötigt wird, um das Dampfvolumen zu erzeugen. Dadurch wird sichergestellt, dass dem Verdampfer 6 nur die Menge an Fluid 14 zugeleitet wird, welche benötigt wird, um das Atemgas zu konditionieren. Durch diese Ansteuerung wird vermieden, dass zu große oder zu geringe Mengen an Fluid 14 dem Verdampfer 6 zugeleitet werden. Dies wirkt einer unzureichenden Konditionierung und einer Übersättigung des Atemgases entgegen. Dadurch wird der Patient 12 optimal versorgt und eine Kondensation des Atemgases innerhalb der Atemgaszufuhr 2 wird vermieden.

Die Steuerungseinheit 11 kann die Information des Beatmungsgerätes 1 oder des Sensors 10 in Echtzeit verarbeiten und das Atemgas-Anfeuchtungssystem 5 in Echtzeit steuern. Durch diesen Echtzeitbetrieb des Atemgas-Anfeuchtungssystems kann die Steuerungseinheit 11 besonders schnell auf die aktuelle und individuelle Atemtätigkeit des Patienten 12 reagieren. Insbesondere kann die Steuerungseinheit 11 auf eine unruhige Atmung des Patienten 12 reagieren und somit eine effektive Versorgung des Patienten 12 sicherstellen.

Die Pumpe 6 und der Verdampfer 7 sind vorzugsweise nur während der Einatmung des Patienten 12 aktiv. Hierdurch kann sichergestellt werden, dass während der Ausatmung kein Fluid 14 verdampft wird und es zu einer Übersättigung des Atemgases innerhalb des Verdampfers 6 und/oder der Atemgaszufuhr 2 kommt und das Atemgas kondensiert.

Das Fluid 14, welches die Pumpe 7 dem Verdampfer 6 zuleitet, wird durch den Verdampfer 6 vollständig verdampft. Somit verbleibt kein Fluid 14 im Verdampfer 6. Dies hat den Vorteil, dass während der nächsten Einatmung des Patienten 12 die richtige Menge an Fluid 14 im Verdampfer 6 vorliegt. Dadurch wird sichergestellt, dass sich kein Fluid 14 im Verdampfer 6 ansammelt.

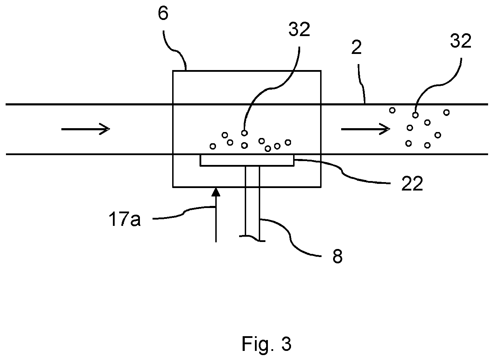

Wie in Fig. 3 dargestellt, umfasst der Verdampfer 6 ein Heizelement 22, welches vorzugsweise ein Peltier-Element ist. Das Heizelement 22 ist direkt an der Atemgaszufuhr 2 positioniert, sodass das Fluid 14 direkt in der Atemgaszufuhr 2 von dem Heizelement 22 erhitzt und verdampft werden kann. Durch die Verdampfung entstehen Aerosolpartikel 32, welche direkt mit dem Atemgas in Berührung kommen. Die Aerosolpartikel 32 befeuchten das Atemgas und werden mit dem Atemgas zusammen vom Patienten 12 eingeatmet.

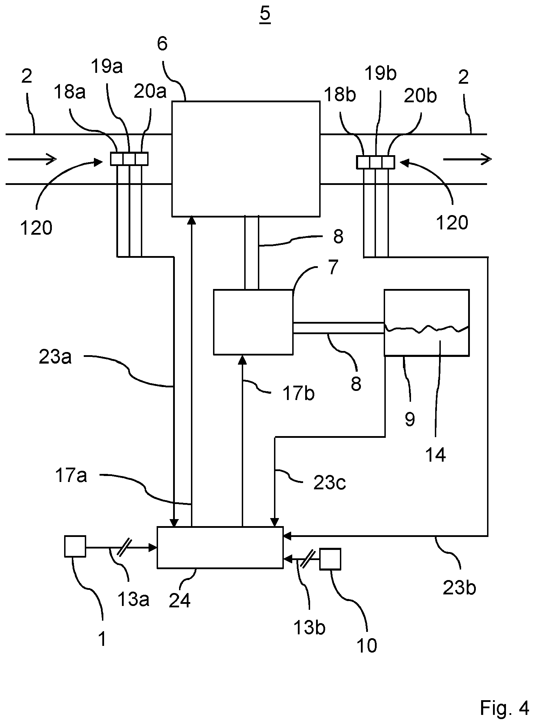

Eine zweite Ausgestaltung des Atemgas-Anfeuchtungssystems 5 ist in Fig. 4 dargestellt. Hierbei umfasst das Atemgas-Anfeuchtungssystem 5 ein zweites Sensorsystem 120. Das zweite Sensorsystem 120 ist innerhalb der Atemgaszufuhr 2 positioniert und umfasst mehrere Sensoren, welche vor und nach dem Verdampfer 6 angeordnet sind. Alternativ können die Sensoren entweder ausschließlich vor oder nach dem Verdampfer 6 angeordnet sein.

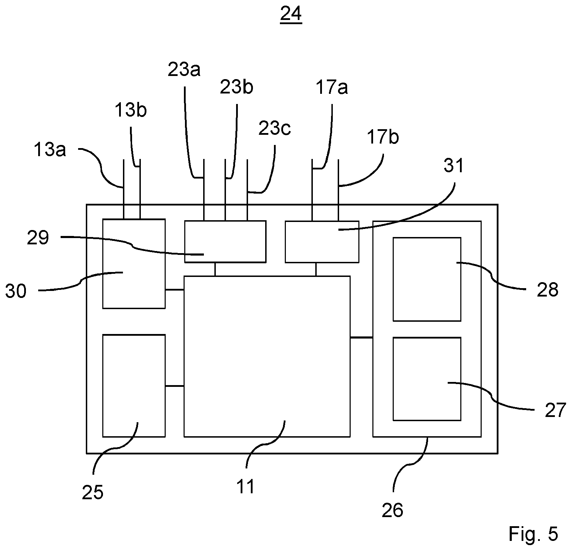

Die Sensoren des zweiten Sensorsystems 120, welche sich vor dem Verdampfer 6 befinden, sind über eine Messsignalleitung 23a mit dem Computer 24 verbunden. Die Sensoren, welche nach dem Verdampfer 6 positioniert sind, werden über eine Messsignalleitung 23b mit dem Computer 24 verbunden. Die Messsignalleitungen 23a, 23b werden über die Sensorschnittstelle 29 des Computers 24 mit der Steuerungseinheit 11 verbunden, wie Fig. 5 zeigt.

Bei den Sensoren des zweiten Sensorsystems 120 handelt es sich z. B. um Feuchtigkeitssensoren 18a, 18b. Mit den Feuchtigkeitssensoren 18a, 18b wird vorzugsweise die relative Feuchte des Atemgases vor und nach dem Verdampfer 6 bestimmt. Durch eine Messung der Feuchte durch den Feuchtigkeitssensor 18a vor dem Verdampfer 6 kann bestimmt werden, wie viel Feuchtigkeit und somit Fluiddampf dem Atemgas hinzugefügt werden muss. Durch die Messung der Feuchte durch den Feuchtigkeitssensor 18b nach dem Verdampfer 6 kann festgestellt werden, wie erfolgreich die Konditionierung des Atemgases mit Fluiddampf ist.

Ferner kann das Atemgas-Anfeuchtungssystem 5 Temperatursensoren 19a, 19b umfassen. Je höher die Temperatur des Atemgases ist, desto mehr Fluid 14 kann in diesem gelöst werden. Durch die Messung der Temperatur mittels des Temperatursensors 19a vor dem Verdampfer 6 kann somit bestimmt werden, wie viel Fluid 14 dem Verdampfer zugeführt werden muss, um eine optimale Konditionierung zu erreichen. Hierzu können zusätzlich die Sensordaten der Feuchtigkeitssensoren 18a, 18b herangezogen werden. Durch eine Messung der Temperatur durch den Temperatursensor 19b nach dem Verdampfer kann überprüft werden, ob die gewünschte Temperatur des Atemgases erreicht wird.

Des Weiteren kann das Atemgas-Anfeuchtungssystem 5 Strömungssensoren 20a, 20b umfassen. Hiermit kann beispielsweise die aktuelle Strömungsgeschwindigkeit des Atemgases innerhalb der Atemgaszufuhr 2 bestimmt werden und die Leistung des Verdampfers 6 und der Pumpe 7 entsprechend angepasst werden.

Zusätzlich oder alternativ kann das zweite Sensorsystem 120 beispielsweise Sensoren zur Bestimmung der Atemgaszusammensetzung oder Drucksensoren umfassen.

Die Steuerungseinheit 11 kann die Sensordaten des zweiten Sensorsystems 120 verwenden, um die Leistung des Atemgas-Anfeuchtungssystems 5 so zu steuern, dass eingestellte Zielgrößen erreicht werden. Die Eigenschaften des Atemgases nach dem Verdampfer 6 kann über die Sensoren des zweiten Sensorsystems 120 gemessen werden. Die gemessenen Eigenschaften können mit den eingestellten Zielgrößen verglichen werden. Dadurch kann die Steuerungseinheit 11 die Leistung des Atemgas-Anfeuchtungssystems 5 so anpassen, dass die Zielgrößen erreicht werden. Das Erreichen der Zielgrößen kann durch die fortlaufende Messung der Eigenschaften laufend kontrolliert werden und die Leistung des Atemgas-Anfeuchtungssystems 5 kontinuierlich angepasst werden.

Ferner können die Sensordaten des zweiten Sensorsystems 120 dazu verwendet werden, das benötigte Dampfvolumen zur Konditionierung des Atemgases zu bestimmen. Durch die Analyse des Atemgases vor dem Verdampfer 6 können die Eigenschaften des nicht-konditionierten Atemgases gemessen werden. Damit kennt die Steuerungseinheit 11 die Eigenschaften des Atemgases vor der Konditionierung. In Kombination mit der Information des Sensors 10 oder des Beatmungsgerätes 1 kann die Steuerungseinheit 11 die Leistung des Atemgas-Anfeuchtungssystems 5 an die aktuelle und individuelle Atemtätigkeit des Patienten 12 und die Eigenschaften des Atemgases vor dem Verdampfer 6 angepasst werden. Dadurch können Schwankungen des Feuchtigkeitsgrades und der Temperatur des Atemgases ausgeglichen werden.

Durch eine Überprüfung der Eigenschaften des konditionierten Atemgases mit Hilfe des zweiten Sensorsystems 120 kann die Leistung des Atemgasanfeuchtungssystems noch präziser an den Bedarf des Patienten angepasst werden.

Die relative Feuchte kann durch das Atemgas-Anfeuchtungssystem 5 auf einen Wert zwischen 80 % und 100 %, vorzugsweise zwischen 90 % und 100 %, besonders bevorzugt zwischen 95 % und 100 % konditioniert werden. Das Atemgas-Anfeuchtungssystem kann das Atemgas auf eine Temperatur von 33 °C bis 38 °C, vorzugsweise von 35 °C bis 37 °C, besonders bevorzugt von 36 °C bis 37 °C temperieren. Dadurch kann der Patient 12 bestmöglich versorgt und die Reinigungsmechanismen des Atemtraktes 21, z. B. die mukoziliäre Clearance, kann gefördert werden. Hierdurch wird die Gefahr eine Keimbesiedlung innerhalb des Atmungstraktes 21 wirksam reduziert.

### BEZUGSZEICHENLISTE

- 1: Beatmungsgerät
- 2: Atemgaszufuhr
- 3: Atemgasrückfuhr
- 4: Y-Stück
- 5: Atemgas-Anfeuchtungssystem
- 6: Verdampfer
- 7: Pumpe
- 8: Fluidleitung
- 9: Fluidreservoir
- 10: Sensor
- 11: Steuerungseinheit
- 12: Patient
- 13a: Datenleitung
- 13b: Datenleitung
- 14: Fluid
- 15: Zwerchfell
- 16: Lunge
- 17a: Steuerungssignalleitung
- 17b: Steuerungssignalleitung
- 18a: Feuchtigkeitssensor
- 18b: Feuchtigkeitssensor
- 19a: Temperatursensor
- 19b: Temperatursensor
- 20a: Strömungssensor
- 20b: Strömungssensor
- 21: Atemtrakt
- 22: Heizelement
- 23a: Messsignalleitung
- 23b: Messsignalleitung
- 23c: Messsignalleitung
- 24: Computer
- 25: Energieversorgung
- 26: Benutzerschnittstelle
- 27: Display
- 28: Bedienelement
- 29: Sensorschnittstelle
- 30: Signalauswertung
- 31: Steuerungssignalausgang
- 32: Aerosolpartikel
- 33: Anschlussstück
- 100: Sensorsystem
- 120: Sensorsystem

## Patentansprüche

1. Atemgas-Anfeuchtungssystem (5) zur Konditionierung von Atemgas bei der Beatmung von Patienten (12) umfassend
ein Fluidreservoir (9), nämlich ein Wasserreservoir, zur Bereitstellung eines Fluidvorrats,
einen Verdampfer (6) zur Verdampfung eines zugeführten Fluids (14) in Verbindung mit einer zum Patienten (12) führenden Atemgaszufuhr (2), wobei durch die Verdampfung des zugeführten Fluids (14) das Atemgas hinsichtlich der Temperatur und der Feuchte konditioniert wird,
eine Pumpe (7) für den Transport einer definierten Menge an Fluid (14) von dem Fluidreservoir (9) zu dem Verdampfer (6),
ein erstes Sensorsystem (100) mit mindestens einem, vorzugsweise mehreren Sensoren zur Ermittlung der Atemtätigkeit des Patienten (12),
eine Steuerungseinheit (11) zur Steuerung des Atemgas-Anfeuchtungssystems (5),
wobei der Verdampfer (6) ein Heizelement (22) umfasst, und
die Pumpe (7) dem Verdampfer (6) eine von der Atemtätigkeit des Patienten (12) abhängige Menge an Fluid (14) zuleitet, der Verdampfer (6) diese Menge verdampft und dem Patienten das angefeuchtete Atemgas zugeführt wird,
das erste Sensorsystem (100) einen Sensor (10) für eine Information zur Spontanatmung des Patienten (12) umfasst und die Steuerungseinheit (11) die Information zur Spontanatmung des Patienten (12) erhält oder
die Steuerungseinheit (11) eine Information zur Beatmungstätigkeit eines Beatmungsgerätes (1) erhält,
von der Pumpe (7) in Abhängigkeit der jeweiligen Information eine Fluidmenge dosiert und vom Verdampfer (6) verdampft wird, und
der Verdampfer (6) das durch die Pumpe (7) zugeleitete Fluid (14) vollständig verdampft, sodass kein Fluid (14) mehr im Verdampfer (6) verbleibt.

2. . Atemgas-Anfeuchtungssystem (5) nach Anspruch 1, **dadurch gekennzeichnet, dass** der Sensor (10) am Patienten (12) angeordnet ist.

3. . Atemgas-Anfeuchtungssystem (5) nach Anspruch 1 oder 2, **dadurch gekennzeichnet, dass** der Sensor (10) ein Abdominalsensor zur Messung der Abdominalbewegung des Patienten (12) und/oder ein Zwerchfellsensor zur Messung der Zerchfellbewegung des Patienten (12) ist.

4. . Atemgas-Anfeuchtungssystem (5) nach einem der vorhergehenden Ansprüche, **dadurch gekennzeichnet, dass** die Steuerungseinheit (11) erkennt, ob sie die Information von dem Beatmungsgerät (1) oder von dem Sensor (10) erhält.

5. . Atemgas-Anfeuchtungssystem (5) nach einem der vorhergehenden Ansprüche, **dadurch gekennzeichnet, dass** in Abhängigkeit der Information das benötigte Dampfvolumen bestimmt wird.

6. . Atemgas-Anfeuchtungssystem (5) nach Anspruch 5, **dadurch gekennzeichnet, dass** die Pumpe (7) von der Steuerungseinheit (11) in der Art ansteuerbar ist, dass die Pumpe (7) dem Verdampfer (6) eine bestimmte Menge an Fluid (14) zuleitet, welches benötigt wird, um das benötigte Dampfvolumen durch den Verdampfer (6) zu erzeugen.

7. . Atemgas-Anfeuchtungssystem (5) nach einem der vorhergehenden Ansprüche, **dadurch gekennzeichnet, dass** die Steuerungseinheit (11) die Information in Echtzeit verarbeitet und das Atemgas-Anfeuchtungssystem (5) in Echtzeit ansteuerbar ist.

8. . Atemgas-Anfeuchtungssystem (5) nach einem der vorhergehenden Ansprüche, **dadurch gekennzeichnet, dass** die Pumpe (7) und der Verdampfer (6) nur für die Einatmung des Patienten (12) aktiviert sind.

9. . Atemgas-Anfeuchtungssystem (5) nach einem der vorhergehenden Ansprüche, **dadurch gekennzeichnet, dass** das Heizelement (22) ein Peltier-Element ist.

10. . Atemgas-Anfeuchtungssystem (5) nach einem der vorhergehenden Ansprüche, **dadurch gekennzeichnet, dass** das Atemgas-Anfeuchtungssystem (5) ein zweites Sensorsystem (120) mit mindestens einem Sensor, vorzugsweise mehreren Sensoren, umfasst, wobei der Sensor/die Sensoren in der zum Patienten (12) führenden Atemgaszufuhr (2) vor und/oder nach dem Verdampfer (6) positioniert ist/sind.

11. . Atemgas-Anfeuchtungssystem (5) nach Anspruch 10, **dadurch gekennzeichnet, dass** das zweite Sensorsystem (120) mindestens einen Feuchtigkeitssensor (18a, 18b) zur Bestimmung der Feuchte des zugeführten Atemgases umfasst.

12. . Atemgas-Anfeuchtungssystem (5) nach Anspruch 10, **dadurch gekennzeichnet, dass** das zweite Sensorsystem (120) mindestens einen Temperatursensor (19a, 19b) zur Bestimmung der Temperatur des zugeführten Atemgases umfasst.

13. . Atemgas-Anfeuchtungssystem (5) nach Anspruch 10, **dadurch gekennzeichnet, dass** das zweite Sensorsystem (120) mindestens einen Strömungssensor (20a, 20b) zur Bestimmung der Strömungsgeschwindigkeit des zugeführten Atemgases umfasst.

14. . Atemgas-Anfeuchtungssystem (5) nach einem der vorhergehenden Ansprüche 10 - 13, **dadurch gekennzeichnet, dass** die Steuerungseinheit (11) die Sensordaten des zweiten Sensorsystems (120) verwendet, um die Leistung des Atemgas-Anfeuchtungssystems (5) zu steuern, sodass eingestellte Zielgrößen erreicht werden.

15. . Atemgas-Anfeuchtungssystem (5) nach Anspruch 5 und einem der vorhergehenden Ansprüche 10 - 14, **dadurch gekennzeichnet, dass** die Steuerungseinheit (11) zur Bestimmung des benötigten Dampfvolumens zusätzlich die Daten des zweiten Sensorsystems (120) verwendet.

16. . Atemgas-Anfeuchtungssystem (5) nach einem der vorhergehenden Ansprüche, **dadurch gekennzeichnet, dass** das Atemgas-Anfeuchtungssystem (5) das Atemgas auf eine relative Feuchte von 80 % bis 100 %, vorzugsweise von 90 % bis 100 %, besonders bevorzugt von 95 % bis 100 %, konditioniert.

17. . Atemgas-Anfeuchtungssystem (5) nach einem der vorhergehenden Ansprüche, **dadurch gekennzeichnet, dass** das Atemgas-Anfeuchtungssystem (5) das Atemgas auf eine Temperatur von 33 °C bis 38 °C, vorzugsweise von 35 °C bis 37 °C, besonders bevorzugt von 36 °C bis 37 °C, konditioniert.

## Claims

1. Respiratory gas humidification system (5) for conditioning respiratory gas when ventilating patients (12), comprising
a fluid reservoir (9), specifically a water reservoir, for providing a fluid storage,
an evaporator (6) for evaporating a supplied fluid (14) in conjunction with a respiratory gas supply (2) leading to the patient (12), the respiratory gas being conditioned in respect of the temperature and the humidity by evaporating the supplied fluid (14),
a pump (7) for transporting a defined amount of fluid (14) from the fluid reservoir (9) to the evaporator (6),
a first sensor system (100) having at least one sensor, preferably a plurality of sensors, for determining the respiratory activity of the patient (12),
a control unit (11) for controlling the respiratory gas humidification system (5),
wherein the evaporator (6) comprises a heating element (22), and
the pump (7) feeds an amount of fluid (14) dependent on the respiratory activity of the patient (12) to the evaporator (6), the evaporator (6) evaporates this amount, and the humidified respiratory gas is supplied to the patient,
the first sensor system (100) comprises a sensor (10) for information regarding the spontaneous respiration of the patient (12), and the control unit (11) receives the information regarding the spontaneous respiration of the patient (12) or
the control unit (11) receives information regarding the ventilation activity of a ventilator (1),
depending on the respective information, an amount of fluid is metered by the pump (7) and evaporated by the evaporator (6), and
the evaporator (6) completely evaporates the fluid (14) fed by the pump (7), with the result that no fluid (14) remains in the evaporator (6) anymore.

2. Respiratory gas humidification system (5) according to Claim 1, **characterized in that** the sensor (10) is arranged on the patient (12).

3. Respiratory gas humidification system (5) according to Claim 1 or 2, **characterized in that** the sensor (10) is an abdominal sensor for measuring the abdominal movement of the patient (12) and/or a diaphragm sensor for measuring the diaphragm movement of the patient (12).

4. Respiratory gas humidification system (5) according to any of the preceding claims, **characterized in that** the control unit (11) recognizes whether it obtains the information from the ventilator (1) or from the sensor (10) .

5. Respiratory gas humidification system (5) according to any of the preceding claims, **characterized in that** the vapour volume required is determined on the basis of the information.

6. Respiratory gas humidification system (5) according to Claim 5, **characterized in that** the pump (7) is driveable by the control unit (11) in such a way that the pump (7) feeds a certain amount of fluid (14) to the evaporator (6), the fluid being required for the evaporator (6) to generate the required vapour volume.

7. Respiratory gas humidification system (5) according to any of the preceding claims, **characterized in that** the control unit (11) processes the information in real time, and the respiratory gas humidification system (5) is controllable in real time.

8. Respiratory gas humidification system (5) according to any of the preceding claims, **characterized in that** the pump (7) and the evaporator (6) are activated only for the inspiration by the patient (12).

9. Respiratory gas humidification system (5) according to any of the preceding claims, **characterized in that** the heating element (22) is a Peltier element.

10. Respiratory gas humidification system (5) according to any of the preceding claims, **characterized in that** the respiratory gas humidification system (5) comprises a second sensor system (120) having at least one sensor, preferably a plurality of sensors, the sensor/sensors being positioned upstream and/or downstream of the evaporator (6) in the respiratory gas supply (2) leading to the patient (12).

11. Respiratory gas humidification system (5) according to Claim 10, **characterized in that** the second sensor system (120) comprises at least one humidity sensor (18a, 18b) for determining the humidity of the supplied respiratory gas.

12. Respiratory gas humidification system (5) according to Claim 10, **characterized in that** the second sensor system (120) comprises at least one temperature sensor (19a, 19b) for determining the temperature of the supplied respiratory gas.

13. Respiratory gas humidification system (5) according to Claim 10, **characterized in that** the second sensor system (120) comprises at least one flow sensor (20a, 20b) for determining the flow speed of the supplied respiratory gas.

14. Respiratory gas humidification system (5) according to any of preceding Claims 10-13, **characterized in that** the control unit (11) uses the sensor data from the second sensor system (120) to control the performance of the respiratory gas humidification system (5) such that set target variables are attained.

15. Respiratory gas humidification system (5) according to Claim 5 and any of preceding Claims 10-14, **characterized in that** the control unit (11) additionally uses the data from the second sensor system (120) to determine the vapour volume required.

16. Respiratory gas humidification system (5) according to any of the preceding claims, **characterized in that** the respiratory gas humidification system (5) conditions the respiratory gas to a relative humidity of 80% to 100%, preferably 90% to 100%, particularly preferably 95% to 100%.

17. Respiratory gas humidification system (5) according to any of the preceding claims, **characterized in that** the respiratory gas humidification system (5) conditions the respiratory gas to a temperature of 33°C to 38°C, preferably 35°C to 37°C, particularly preferably 36°C to 37°C.

## Revendications

1. Système d'humidification de gaz respiratoire (5) permettant de conditionner un gaz respiratoire lors de la ventilation de patients (12), comprenant
un réservoir de fluide (9), notamment un réservoir d'eau, pour fournir une réserve de fluide,
un vaporisateur (6) pour vaporiser un fluide alimenté (14) en relation avec une alimentation de gaz respiratoire (2) menant au patient (12), dans lequel la vaporisation du fluide alimenté (14) conditionne le gaz respiratoire en ce qui concerne la température et l'humidité,
une pompe (7) pour le transport d'une quantité définie de fluide (14) du réservoir de fluide (9) au vaporisateur (6),
un premier système de capteur (100) avec au moins un, de préférence plusieurs capteurs, pour établir l'activité respiratoire du patient (12),
une unité de commande (11) pour commander le système d'humidification de gaz respiratoire (5),
dans lequel le vaporisateur (6) comprend un élément chauffant (22), et
la pompe (7) amène au vaporisateur (6) une quantité de fluide (14) dépendant de l'activité respiratoire du patient (12), le vaporisateur (6) vaporise cette quantité, et le gaz respiratoire humidifié est alimenté au patient,
le premier système de capteur (100) comprend un capteur (10) pour une information sur la respiration spontanée du patient (12), et l'unité de commande (11) reçoit l'information sur la respiration spontanée du patient (12), ou
l'unité de commande (11) reçoit une information sur l'activité de ventilation d'un appareil respiratoire (1),
la pompe (7) dose une quantité de fluide en fonction de l'information respective et le vaporisateur (6) la vaporise, et
le vaporisateur (6) vaporise complètement le fluide (14) amené par la pompe (7) de sorte que plus aucun fluide (14) ne reste dans le vaporisateur (6).

2. Système d'humidification de gaz respiratoire (5) selon la revendication 1, **caractérisé en ce que** le capteur (10) est disposé au niveau du patient (12).

3. Système d'humidification de gaz respiratoire (5) selon la revendication 1 ou 2, **caractérisé en ce que** le capteur (10) est un capteur abdominal pour mesurer le mouvement abdominal du patient (12) et/ou un capteur de diaphragme pour mesurer le mouvement de diaphragme du patient (12).

4. Système d'humidification de gaz respiratoire (5) selon l'une quelconque des revendications précédentes, **caractérisé en ce que** l'unité de commande (11) reconnaît si elle reçoit l'information de l'appareil respiratoire (1) ou du capteur (10).

5. Système d'humidification de gaz respiratoire (5) selon l'une quelconque des revendications précédentes, **caractérisé en ce que** le volume de vapeur nécessaire est déterminé en fonction de l'information.

6. Système d'humidification de gaz respiratoire (5) selon la revendication 5, **caractérisé en ce que** la pompe (7) peut être pilotée par l'unité de commande (11) de telle sorte que la pompe (7) amène une quantité déterminée de fluide (14) au vaporisateur (6), qui est nécessaire pour produire le volume de vapeur nécessaire à l'aide du vaporisateur (6).

7. Système d'humidification de gaz respiratoire (5) selon l'une quelconque des revendications précédentes, **caractérisé en ce que** l'unité de commande (11) traite l'information en temps réel, et le système d'humidification de gaz respiratoire (5) peut être piloté en temps réel.

8. Système d'humidification de gaz respiratoire (5) selon l'une quelconque des revendications précédentes, **caractérisé en ce que** la pompe (7) et le vaporisateur (6) ne sont activés que pour l'inhalation du patient (12) .

9. Système d'humidification de gaz respiratoire (5) selon l'une quelconque des revendications précédentes, **caractérisé en ce que** l'élément chauffant (22) est un élément Peltier.

10. Système d'humidification de gaz respiratoire (5) selon l'une quelconque des revendications précédentes, **caractérisé en ce que** le système d'humidification de gaz respiratoire (5) comprend un deuxième système de capteur (120) avec au moins un capteur, de préférence plusieurs capteurs, dans lequel le(s) capteur(s) est/sont positionné(s) dans l'alimentation de gaz respiratoire (2) menant au patient (12), avant et/ou après le vaporisateur (6).

11. Système d'humidification de gaz respiratoire (5) selon la revendication 10, **caractérisé en ce que** le deuxième système de capteur (120) comprend au moins un capteur d'humidité (18a, 18b) pour déterminer l'humidité du gaz respiratoire alimenté.

12. Système d'humidification de gaz respiratoire (5) selon la revendication 10, **caractérisé en ce que** le deuxième système de capteur (120) comprend au moins un capteur de température (19a, 19b) pour déterminer la température du gaz respiratoire alimenté.

13. Système d'humidification de gaz respiratoire (5) selon la revendication 10, **caractérisé en ce que** le deuxième système de capteur (120) comprend au moins un capteur de débit (20a, 20b) pour déterminer la vitesse de débit du gaz respiratoire alimenté.

14. Système d'humidification de gaz respiratoire (5) selon l'une quelconque des revendications précédentes 10 à 13, **caractérisé en ce que** l'unité de commande (11) utilise les données de capteur du deuxième système de capteur (120) pour commander la puissance du système d'humidification de gaz respiratoire (5) de façon à atteindre des grandeurs cibles réglées.

15. Système d'humidification de gaz respiratoire (5) selon la revendication 5 et l'une des revendications précédentes 10 à 14, **caractérisé en ce que** l'unité de commande (11) utilise en plus les données du deuxième système de capteur (120) pour déterminer le volume de vapeur nécessaire.

16. Système d'humidification de gaz respiratoire (5) selon l'une quelconque des revendications précédentes, **caractérisé en ce que** le système d'humidification de gaz respiratoire (5) conditionne le gaz respiratoire à une humidité relative de 80 % à 100 %, de préférence de 90 % à 100 %, de manière particulièrement préférée de 95 % à 100 %.

17. Système d'humidification de gaz respiratoire (5) selon l'une quelconque des revendications précédentes, **caractérisé en ce que** le système d'humidification de gaz respiratoire (5) conditionne le gaz respiratoire à une température de 33 °C à 38 °C, de préférence de 35 °C à 37 °C, de manière particulièrement préférée de 36 °C à 37 °C.
